# EUROPEAN PATENT APPLICATION

(11) **EP 4 796 029 A1**
(43) Date of publication of application: **26.08.2026**
(21) Application number: 25219298.4
(22) Date of filing: 28.11.2025
(51) Int. Cl.: A61B 5/145

(54) **APPLICATOR ASSEMBLY**

(30) Priority: 21.02.2025 KR 20250023037
(71) Applicant: I-SENS, INC., Seoul 06646 (KR)
(72) Inventor: CHOI, Hyun Ho, 06646 Seoul (KR)
(74) Representative: BCKIP Part mbB

(57) **Abstract**

An applicator assembly including a sensor unit configured to be attached to a user's body to acquire the user's biometric information, an applicator including a case forming an interior space with an opening formed on one side, configured to attach the sensor unit to the user's body, and a protective cap configured to be coupled to the case to shield the opening, in which the applicator includes a sealing member disposed adjacent to the opening of the case and configured to seal the interior space of the case together with the protective cap, and the sealing member includes a first protrusion and a second protrusion that protrude outward from a surface of the sealing member and form a step relative to each other is provided.

## Description

### CROSS-REFERENCE TO RELATED APPLICATION(S)

This application claims the benefit of Korean Patent Application No. 10-2025-0023037, filed on February 21, 2025, in the Korean Intellectual Property Office.

### FIELD OF THE INVENTION

Example embodiments relate to an applicator assembly, and more particularly, to an applicator assembly for acquiring biometric information.

### BACKGROUND

Modem people suffer from various diseases, and to properly treat or manage these diseases, means of acquiring human biometric information are necessary.

Diabetes is a prime example. To properly manage diabetes, means of acquiring blood glucose information, which is the biometric data of diabetic patients, are required.

Recently, continuous glucose monitoring (CGM) devices have been developed that are inserted into the human body and measure blood glucose levels at intervals of several minutes. CGM devices minimize the pain and discomfort associated with blood sampling by inserting a needle-like sensor into relatively painless areas like the abdomen or arm, enabling continuous glucose measurement.

A continuous glucose monitoring device is composed of a sensor unit attached to the user's body to measure the user's blood glucose information and transmit the measured blood glucose information, a terminal that provides the user with the blood glucose information received from the sensor unit, enabling the user to monitor or manage their blood glucose information, and so on.

The sensor unit is composed of a sensor module inserted and attached to the user's skin to measure blood glucose from the user's body fluids, and a transmitter that sends the blood glucose levels measured by the sensor module to the terminal. The sensor module includes a probe, formed in a needle shape, inserted into subcutaneous fat to extract interstitial fluid.

An applicator is used to insert and attach the sensor unit to the body.

The applicator is configured to insert and attach the sensor unit to the body. Upon user operation, it inserts and attaches the sensor unit to the body. When the applicator is subsequently separated and removed from the body while the sensor unit is inserted and attached, only the sensor unit remains attached to the body.

Since a part of the sensor unit is inserted into the human body, the sensor unit installed inside the applicator and the interior space of the applicator must be maintained in a sterile state from the manufacturing stage until use by the consumer. Additionally, a dehumidified state must be maintained to preserve the electronic components. Simultaneously, the interior space of the applicator must be sealed from the outside to maintain its sterile and dehumidified state.

Various packaging technologies are applied to applicators to maintain these sterile, dehumidified, and sealed conditions, and simplifying packaging and reducing size have become one of the goals for applicator manufacturers.

Meanwhile, to maintain the applicator's sealed state, a protective cap is attached to the case, and the user detaches this protective cap from the case for use. These sealing structures must maintain the applicator's sealed integrity until use and be designed to ensure no inconvenience to the user during removal.

### SUMMARY OF THE INVENTION

An aspect is to address at least some of the problems of the prior art described above and provides an applicator with improved sealing integrity.

Another aspect also provides an applicator that reduces the force burden required by the user to detach the protective cap from the applicator assembly.

According to an aspect, there is provided an applicator assembly including a sensor unit configured to be attached to a user's body to acquire the user's biometric information, an applicator including a case forming an interior space with an opening that is opened on one side, configured to attach the sensor unit to the user's body, and a protective cap configured to be coupled to the case to shield the opening. The applicator may include a sealing member disposed adjacent to the opening of the case and configured to seal the interior space of the case together with the protective cap, and the sealing member may include a first protrusion and a second protrusion that protrude outward from a surface of the sealing member and form a step relative to each other.

According to an example embodiment, the protective cap may cover at least a portion of an outer surface of the case adjacent to the opening.

According to an example embodiment, the first protrusion may be positioned farther from the opening than the second protrusion and protrude more than the second protrusion.

According to an example embodiment, the protective cap may include a first sealing surface configured to compress the first protrusion and a second sealing surface configured to compress the second protrusion while being coupled to the case.

According to an example embodiment, the first sealing surface and the second sealing surface may form a step relative to each other, and a distance from the surface of the sealing member to the first sealing surface may be greater than a distance from the surface of the sealing member to the second sealing surface.

According to an example embodiment, the distance from the surface of the sealing member to the first sealing surface may be greater than a height by which the second protrusion protrudes from the surface of the sealing member.

According to an example embodiment, the first protrusion and the second protrusion may include an elastic material capable of compressive deformation, a height by which the first protrusion protrudes from the surface of the sealing member before compression may be greater than the distance from the surface of the sealing member to the first sealing surface, and a height by which the second protrusion protrudes from the surface of the sealing member before compression may be greater than the distance from the surface of the sealing member to the second sealing surface.

According to an example embodiment, the sealing member may further include a band portion configured to cover around an outer surface of the case, and the case may include a recess configured to accommodate the band portion.

According to an example embodiment, the band portion may be configured to be detachably coupled to the case.

According to an example embodiment, the sealing member may be integrally formed with the case from the same material as the case.

According to an example embodiment, the first protrusion and the second protrusion may be configured to be detachably coupled to the case and have a ring shape that covers around an outer surface of the case.

According to an example embodiment, the first protrusion and the second protrusion may have a circular cross-section, and a cross-sectional area of the first protrusion may be formed to be larger than a cross-sectional area of the second protrusion.

According to an example embodiment, the surface of the sealing member may be inclined toward an inside of the case as approaching the opening.

According to an example embodiment, an inner surface of the protective cap facing the surface of the sealing member may be inclined along the inclined surface of the sealing member.

According to an example embodiment, the sealing member may be formed from a material capable of compressive deformation and include at least one of synthetic resin, rubber, or silicone.

Additional aspects of example embodiments will be set forth in part in the description which follows and, in part, will be apparent from the description, or may be learned by practice of the disclosure.

### BRIEF DESCRIPTION OF THE DRAWINGS

These and/or other aspects, features, and advantages of the disclosure will become apparent and more readily appreciated from the following description of example embodiments, taken in conjunction with the accompanying drawings of which:
FIG. 1 is a perspective view showing an applicator assembly according to an example embodiment of the present disclosure;
FIG. 2 is a perspective view showing an applicator according to an example embodiment of the present disclosure and a sensor unit assembled to the applicator as viewed from another direction;
FIG. 3 is an exemplary drawing showing a cross-sectional view along line I-I' in FIG. 1;
FIG. 4 is an exemplary diagram showing an enlarged view of area A in FIG. 3;
FIG. 5 is an exemplary diagram showing an enlarged view of area A in FIG. 3;
FIG. 6 is a diagram illustrating a process of detaching the protective cap from the applicator of FIG. 5;
FIG. 7 is a diagram illustrating a process of detaching the protective cap from the applicator of FIG. 5;
FIG. 8 is an exemplary diagram showing an applicator according to another example embodiment of the present disclosure, enlarged from area A of FIG. 3;
FIG. 9 is an exemplary diagram showing an applicator according to yet another example embodiment of the present disclosure, enlarging from area A of FIG. 3;
FIG. 10 is an illustrative diagram showing an applicator according to yet another example embodiment of the present disclosure, enlarged from area B of FIG. 6;
FIG. 11 is a cross-sectional view showing the state before triggering of an applicator according to an example embodiment of the present disclosure;
FIG. 12 is a cross-sectional view showing the button portion of the applicator in FIG. 10 pressed;
FIG. 13 is a cross-sectional view showing the state after triggering of the applicator of FIG. 10;
FIG. 14 is a perspective view showing the state of an applicator according to an example embodiment of the present disclosure with a protective cap detached;
FIG. 15 is a cross-sectional view of an applicator according to an example embodiment of the present disclosure in the state before triggering, taken along section line II-II' of FIG. 1; and
FIG. 16 is a cross-sectional view showing of the state after triggering of FIG. 14.

### DETAILED DESCRIPTION OF EXEMPLARY EMBODIMENTS

The terms used in describing example embodiments have been selected as general terms that are currently widely used as possible while taking functions in the present disclosure into consideration, but these may vary according to the intention of those skilled in the art, a precedent, the emergence of new technologies, and the like. In addition, in certain cases, there are terms arbitrarily selected by the applicant, and in this case, the meaning will be described in detail in the corresponding description. Therefore, the terms used in the present disclosure may be defined based on the meaning of the term and the whole contents of the present disclosure, not just the name of the term.

The suffix "...part" used for components in this specification is assigned or used interchangeably only for the convenience of drafting the specification and may not inherently denote distinct meanings or functions. Furthermore, when describing example embodiments included in this disclosure, detailed explanations of related prior art may be omitted if deemed to obscure the essence of example embodiments disclosed herein. Furthermore, the accompanying drawings are provided solely to facilitate understanding of example embodiments included in this disclosure. The technical concepts of this disclosure are not limited by the accompanying drawings and should be understood to encompass all modifications, equivalents, and substitutes falling within the scope of the concepts and technical scope of this disclosure.

Terms containing ordinal numbers, such as first, second, etc., may be used to describe various components, but the components are not limited by those terms, and those terms may be used solely for the purpose of distinguishing one component from another.

When a component is described as being "connected" or "coupled to" another component, it may be understood to be directly connected or coupled to that other component, but other components may also exist in between. Conversely, when a component is described as being "directly connected" or "directly coupled" to another component, it may be understood that no other components exist between them.

Unless context clearly indicates otherwise, singular expressions in this specification may include plural expressions.

The terms "comprises," "includes" or "has" as used herein are intended to specify the presence of the features, numbers, steps, actions, components, parts, or combinations thereof described in the specification, and should not be understood as precluding the presence or possibility of one or more other features, numbers, steps, actions, components, parts, or combinations thereof.

The expression "at least one of A, B, and C" as used herein may indicate the following meaning including: A alone; B alone; C alone; both A and B together; both A and C together; both B and C together; or all three of A, B, and C together.

In the following, with reference to the accompanying drawings, example embodiments of the present disclosure will be described in detail so that those of skilled in the art to which the present disclosure pertains may easily implement them. However, the present disclosure may be implemented in various different forms and is not limited to the example embodiments described herein.

Hereinafter, example embodiments of the present disclosure will be described in detail with reference to the drawings.

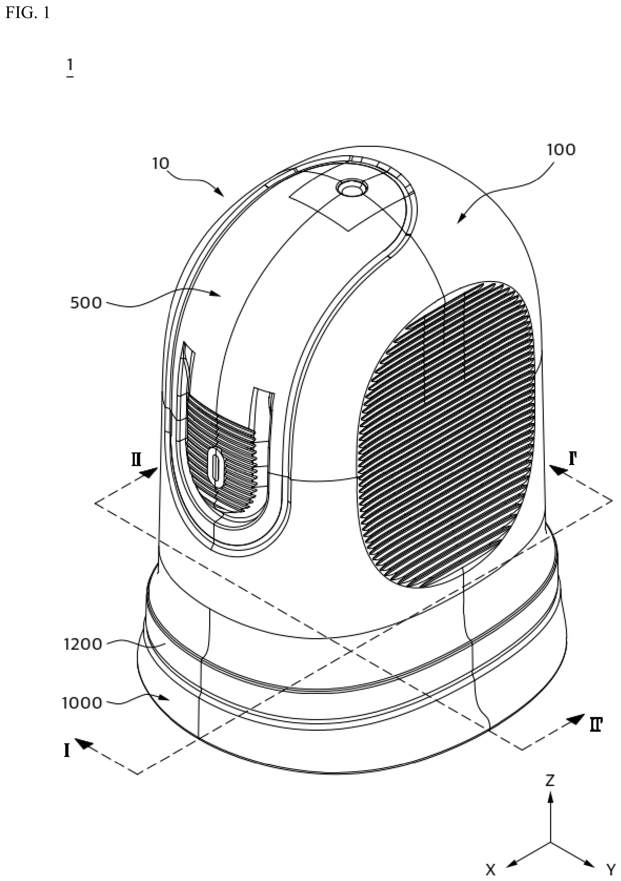

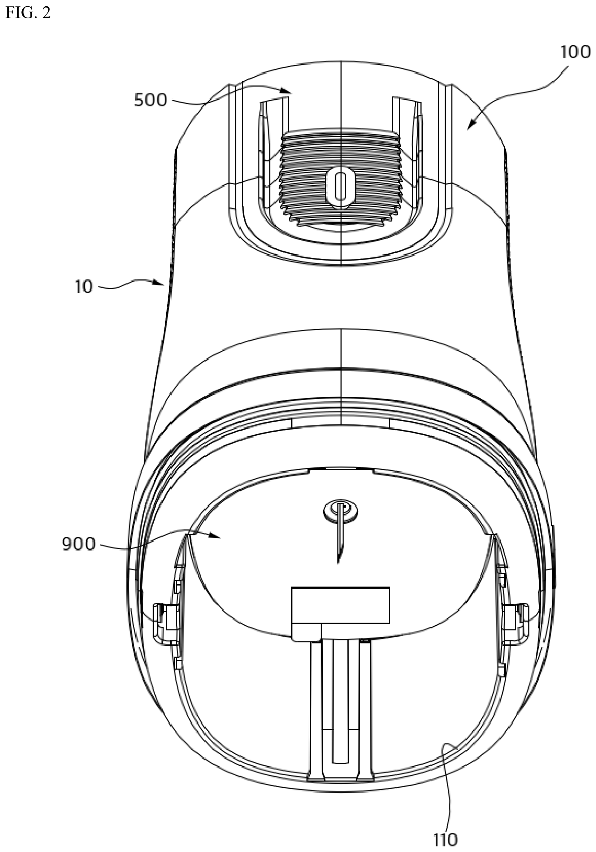

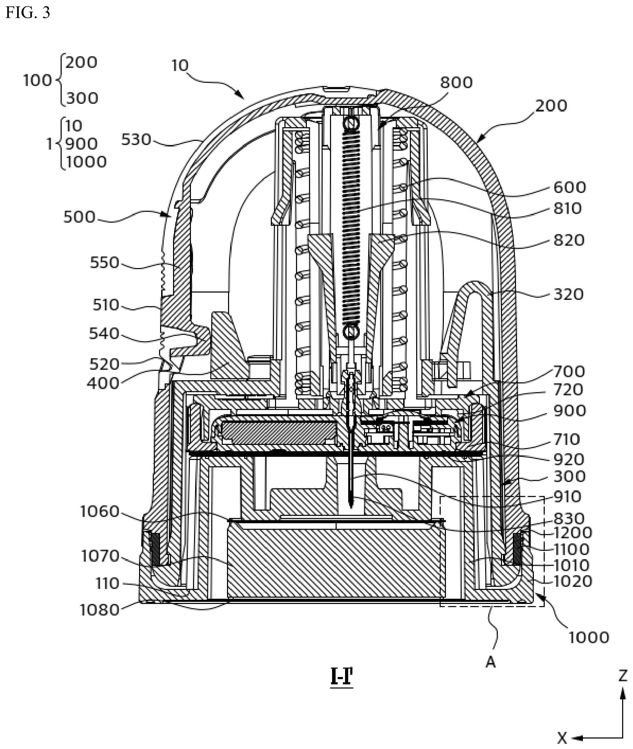

FIG. 1 is a perspective view showing an applicator assembly 1 according to an example embodiment of the present disclosure, FIG. 2 is a perspective view showing an applicator 10 according to an example embodiment of the present disclosure and a sensor unit 900 assembled to the applicator 10 as viewed from another direction, and FIG. 3 is an exemplary diagram showing a cross-sectional view along line I-I' in FIG. 1. FIG. 2 shows the state of the applicator 10 in FIG. 1 with an indicator member 1200 removed and a protective cap 1000 detached.

Referring to FIGS. 1 to 3, the applicator assembly 1 according to an example embodiment of the present disclosure may include the applicator 10. The applicator 10 may be configured to attach the sensor unit 900 to a user's body. For example, the applicator 10 may internally accommodate the sensor unit 900 prior to use and, upon use, trigger the sensor unit 900 to attach the sensor unit 900 to the skin of the user's body.

The applicator 10 according to an example embodiment of the present disclosure may include a case 100.

The case 100 according to an example embodiment of the present disclosure may be a component that protects the components of the applicator 10 and the sensor unit 900 and forms the exterior shape of the applicator 10. The case 100 may form an interior space. The case 100 may form a certain space internally capable of accommodating other components. At this time, the case 100 may have an open side. Alternatively, the case 100 may include an opening 110. The opening 110 may be a portion open on one side of the case 100. The opening 110 may be formed at an end portion of the case 100.

The applicator assembly 1 according to an example embodiment of the present disclosure may include the indicator member 1200. The indicator member 1200 may be adhered between the protective cap 1000 and the case 100. At least a portion of the protective cap 1000 may be coupled to the case 100 such that it covers around the outer surface of the case 100. The indicator member 1200 may surround around an adjacent outer surface between the case 100 and the protective cap 1000 and a gap between the case 100 and the protective cap 1000. At least a portion of the case 100 may include an outwardly protruding protrusion 220. The surface of the protrusion 220 may be flush with the outer surface of the protective cap 1000. The indicator member 1200 may be adhered to the outer surfaces of the protrusion 220 and the protective cap 1000. The indicator member 1200 may be configured to leave a mark on the outer surface of the case 100 or the protective cap 1000 when removed from the case 100 or the protective cap 1000. Therefore, the user may determine whether the sterilization, dehumidification, and sealed state inside the applicator 10 have been compromised by checking whether the indicator member 1200 has been removed. Additionally, the indicator member 1200 may seal the interior of the applicator 10 from the outside by wrapping between the case 100 and the protective cap 1000 in an adhesive manner.

The applicator assembly 1 according to an example embodiment of the present disclosure may include the protective cap 1000. The protective cap 1000 may be coupled to the case 100 of the applicator 10. Specifically, the protective cap 1000 may be detachably coupled to the case 100. The protective cap 1000 may be configured to shield the opening 110 of the case 100. The protective cap 1000 may be coupled in one direction to the case 100 such that the opening 110 is not exposed externally. The user may detach the protective cap 1000 from the case 100 by pulling the protective cap 1000 to one side after removing the indicator member 1200 prior to use.

In an example embodiment of the present disclosure, the protective cap 1000 may be formed to cover at least a portion of the outer peripheral surface of the case 100 adjacent to the opening 110. For example, the protective cap 1000 may be formed to cover at least a portion of the outer peripheral surface of the end portion of the case 100. The protective cap 1000 may have a stopper shape with a concave form to be inserted into the opening 110 of the case 100.

The protective cap 1000 according to an example embodiment of the present disclosure may include a column portion 1010 and an outer wall portion 1020. The column portion 1010 may be a part inserted into the interior of the case 100, and the outer wall portion 1020 may be a part that covers around the outer surface of the case 100. The column portion 1010 may cover around the inner side of the opening 110, and the outer wall portion 1020 may cover around the outer side of the opening 110. For example, a groove may be formed between the column portion 1010 and the outer wall portion 1020 of the protective cap 1000 to allow the end portion of the case 100 to be inserted. The column portion 1010 may cover around the inner side of the end portion of the case 100, and the outer wall portion 1020 may cover around the outer side of the end portion of the case 100.

The protective cap 1000 according to an example embodiment of the present disclosure may include a first cover member 1060, a dehumidifying member 1070, and a second cover member 1080. The first cover member 1060, the dehumidifying member 1070, and the second cover member 1080 may be accommodated inside the protective cap 1000. The protective cap 1000 has holes of various sizes sequentially arranged and penetrated on one side, and the first cover member 1060 and the second cover member 1080 may seal at least one of the holes of various sizes. The dehumidifying member 1070 may be positioned between the first cover member 1060 and the second cover member 1080. The first cover member 1060, the dehumidifying member 1070, and the second cover member 1080 may be arranged sequentially along a single direction.

The first cover member 1060 and the second cover member 1080 according to an example embodiment of the present disclosure may be formed from different materials. The first cover member 1060 may be formed from a material possessing bacterial barrier properties that prevent bacteria from passing through and also possessing breathability. The first cover member 1060 may include a porous filter structure. Therefore, even when sterilizing gas is injected while the first cover member 1060 seals the hole in the protective cap 1000, the sterilizing gas may pass through the first cover member 1060 and be injected into the interior space of the case 100. However, the porous filter structure of the first cover member 1060 may be formed sufficiently small to prevent bacteria from passing through. Therefore, external bacteria may be prevented from passing through the first cover member 1060.

The dehumidifying member 1070 according to an example embodiment of the present disclosure may be a configuration for removing moisture. For example, the dehumidifying member 1070 may include a plurality of desiccants for absorbing moisture.

The second cover member 1080 according to an example embodiment of the present disclosure may be formed from a moisture-proof material. For example, the second cover member 1080 may be an aluminum film. Therefore, external moisture may not be able to pass through the second cover member 1080.

Referring to FIG. 3, the first cover member 1060, dehumidifying member 1070, and second cover member 1080 according to an example embodiment of the present disclosure are accommodated within the protective cap 1000 in a triple-layer structure, enabling the internal environment of the case 100 to be maintained in a sterilized, dehumidified, and sealed state. After sterilizing the interior space of the case 100 by injecting sterilizing gas through the first cover member 1060, the dehumidifying member 1070 is positioned to regulate humidity, and the second cover member 1080 is placed over the outer side to form a structure preventing external bacteria or moisture from penetrating into the interior space of the case 100.

The applicator assembly 1 according to an example embodiment of the present disclosure may include the sensor unit 900. The sensor unit 900 may be configured to acquire the user's biometric information. For example, the sensor unit 900 may be attached to the user's skin S, with at least a portion inserted into the skin S to acquire the user's biometric information. Furthermore, the sensor unit 900 may be configured to transmit the acquired user's biometric information. For example, the sensor unit 900 may transmit the user's biometric information to a portable terminal, allowing the user to check their biometric information via the portable terminal.

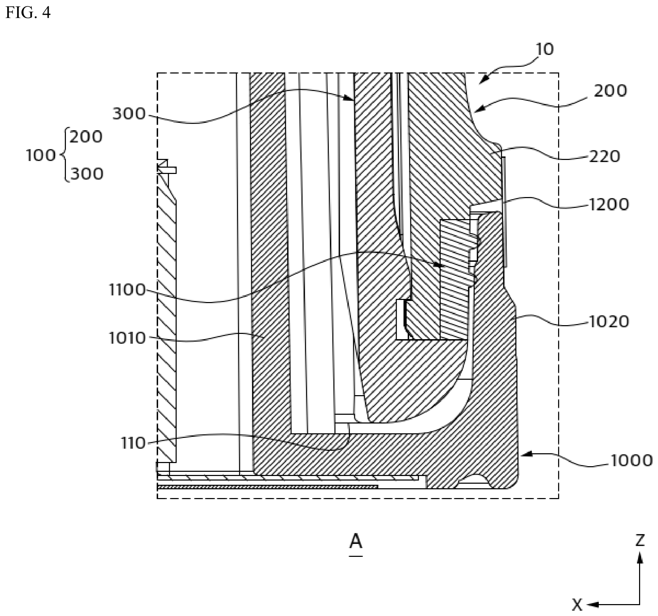

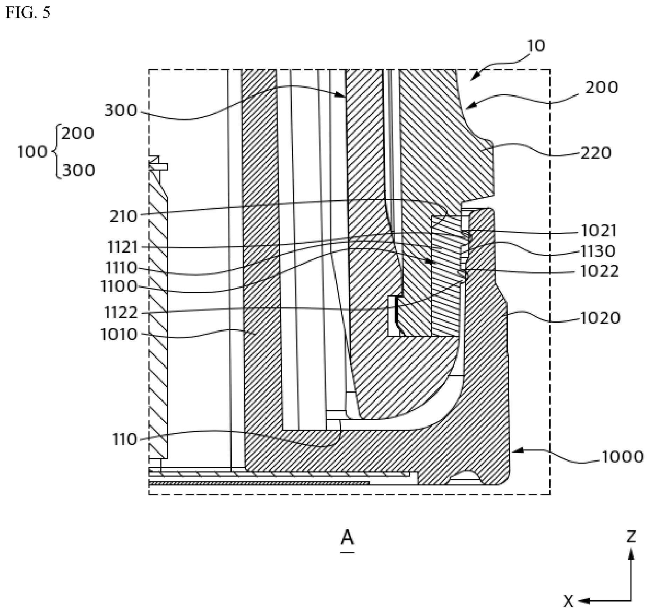

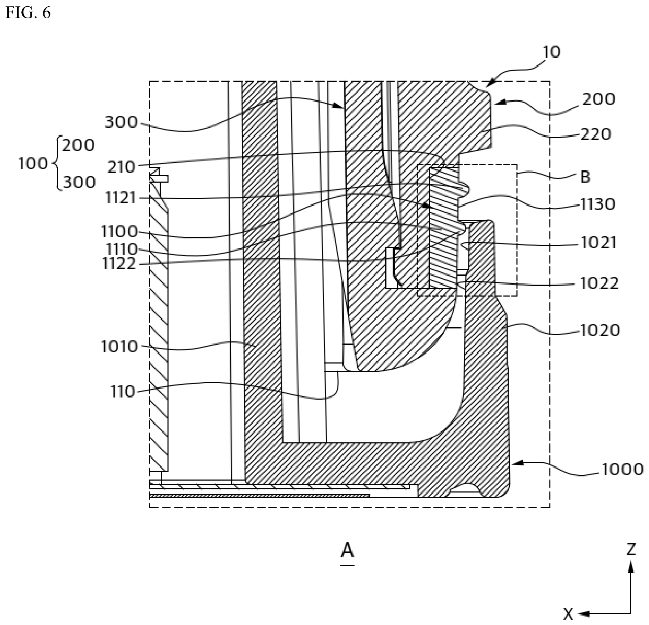

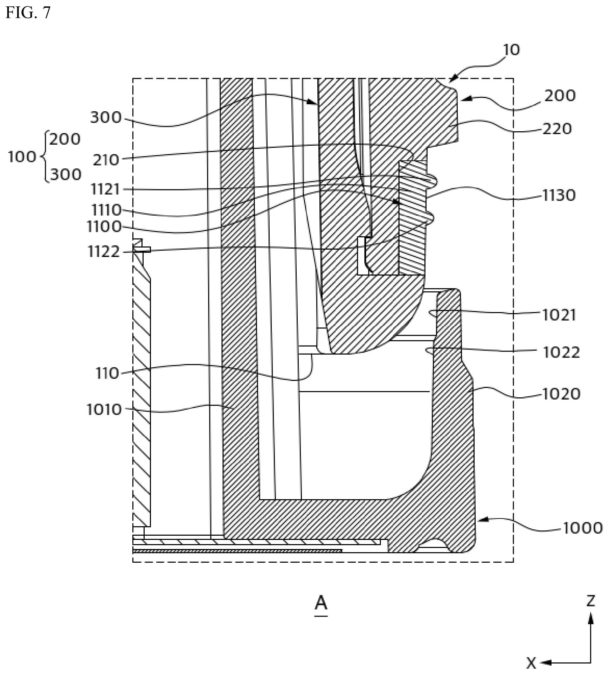

FIG. 4 is an exemplary diagram showing an enlarged view of area A in FIG. 3, FIG. 5 is an exemplary diagram showing an enlarged view of area A in FIG. 3, FIG. 6 is a diagram illustrating a process of detaching the protective cap 1000 from the applicator 10 of FIG. 5, and FIG. 7 is a diagram illustrating a process of detaching the protective cap 1000 from the applicator 10 of FIG. 5. FIGS. 5 to 7 illustrate the state after removing the indicator member 1200 for detaching the protective cap 1000. The protective cap 1000 may be detached in one direction in the sequence from FIG. 5 to FIG. 7.

Referring to FIGS. 4 to 7, the applicator 10 according to an example embodiment of the present disclosure may include a sealing member 1100.

The sealing member 1100 according to an example embodiment of the present disclosure may be positioned adjacent to the opening 110 of the case 100. The sealing member 1100 may be positioned close to the opening 110. The sealing member 1100 may be positioned at a predetermined distance from the opening 110. However, this does not exclude the possibility of the sealing member 1100 being positioned in contact with the opening 110.

The sealing member 1100 according to an example embodiment of the present disclosure may be configured to seal the interior space of the case 100 together with the protective cap 1000. For example, the sealing member 1100 may be disposed between the case 100 and the protective cap 1000 to seal the interior space of the case 100. The sealing member 1100 may fill any void space that may form between the protective cap 1000 and the case 100. Specifically, the outer wall portion 1020 of the protective cap 1000 may contact the sealing member 1100 inwardly. The sealing member 1100 may be positioned between the outer peripheral surface adjacent to the opening 110 of the case 100 and the outer wall portion 1020.

The sealing member 1100 according to an example embodiment of the present disclosure may include a first protrusion 1121 and a second protrusion 1122. The first protrusion 1121 and the second protrusion 1122 may protrude outward from a surface 1130 of the sealing member 1100. The first protrusion 1121 and the second protrusion 1122 may protrude in a direction intersecting the surface 1130 of the sealing member 1100. For example, the first protrusion 1121 and the second protrusion 1122 may protrude toward the protective cap 1000 coupled to the case 100.

The first protrusion 1121 and the second protrusion 1122 according to an example embodiment of the present disclosure may form a step relative to each other. For example, the first protrusion 1121 may protrude more than the second protrusion 1122. Referring to FIGS. 5 to 7, the height by which the first protrusion 1121 protrudes from the surface 1130 of the sealing member 1100 may be greater than the height by which the second protrusion 1122 protrudes.

The first protrusion 1121 according to an example embodiment of the present disclosure may be positioned relatively higher than the second protrusion 1122. In other words, the first protrusion 1121 may be positioned farther from the opening 110 than the second protrusion 1122. In other words, the distance between the opening 110 and the first protrusion 1121 may be greater than the distance between the opening 110 and the second protrusion 1122. Furthermore, the first protrusion 1121 and the second protrusion 1122 may not be physically connected to each other and may maintain different heights.

The protective cap 1000 according to an example embodiment of the present disclosure may include a first sealing surface 1021 and a second sealing surface 1022. When the protective cap 1000 is coupled to the case 100, the first sealing surface 1021 may be configured to compress the first protrusion 1121, and the second sealing surface 1022 may be configured to compress the second protrusion 1122. The first sealing surface 1021 and the second sealing surface 1022 may be the portions that contact the first protrusion 1121 and the second protrusion 1122, respectively, when the protective cap 1000 is coupled to the case 100.

The first protrusion 1121 and the second protrusion 1122 according to an example embodiment of the present disclosure may include an elastic material capable of compressive deformation. For example, the first protrusion 1121 and the second protrusion 1122 may include at least one compressible material such as a synthetic resin, rubber, or silicone.

According to an example embodiment of the present disclosure, the height by which the first protrusion 1121 protrudes from the surface 1130 of the sealing member 1100 before compression may be greater than the distance from the surface 1130 of the sealing member 1100 to the first sealing surface 1021. FIG. 5 is illustrated to explain the aforementioned difference in distance, showing at least a portion of the first protrusion 1121 extending beyond the first sealing surface 1021, which does not depict the actual protective cap 1000 coupled to the case 100 with the first protrusion 1121 in a compressed state. Therefore, the first protrusion 1121 may protrude sufficiently to be compressed by the first sealing surface 1021 and seal the space between the case 100 and the protective cap 1000.

According to an example embodiment of the present disclosure, the height by which the second protrusion 1122 protrudes from the surface 1130 of the sealing member 1100 before compression may be greater than the distance from the surface 1130 of the sealing member 1100 to the second sealing surface 1022. FIG. 5 is illustrated to explain the aforementioned difference in distance, showing at least a portion of the second protrusion 1122 extending beyond the second sealing surface 1022, which does not depict the actual protective cap 1000 coupled to the case 100 with the second protrusion 1122 in a compressed state. Therefore, the second protrusion 1122 may protrude sufficiently to be compressed by the second sealing surface 1022 and seal the space between the case 100 and the protective cap 1000.

The first protrusion 1121 and the second protrusion 1122 according to an example embodiment of the present disclosure may double-seal the applicator 10. The first protrusion 1121 and the second protrusion 1122 may be spaced apart from each other. The first protrusion 1121 and the second protrusion 1122 may seal the space between the case 100 and the protective cap 1000 while being spaced apart. Therefore, even if a defect preventing a smooth seal occurs in the first protrusion 1121, which is located relatively outward from the second protrusion 1122, the second protrusion 1122 may compensate for the sealing integrity.

The first sealing surface 1021 and the second sealing surface 1022 according to an example embodiment of the present disclosure may form a step relative to each other. The second sealing surface 1022 may protrude more toward the sealing member 1100 than the first sealing surface 1021. In other words, the distance from the surface 1130 of the sealing member 1100 to the first sealing surface 1021 may be greater than the distance from the surface 1130 of the sealing member 1100 to the second sealing surface 1022. At this time, the protective cap 1000 may be in a state coupled to the case 100.

The first sealing surface 1021 according to an example embodiment of the present disclosure may be positioned so as not to contact the second protrusion 1122 when the protective cap 1000 is detached. For example, the distance from the surface 1130 of the sealing member 1100 to the first sealing surface 1021 may be greater than the height by which the second protrusion 1122 protrudes from the surface 1130 of the sealing member 1100. Referring to FIGS. 5 to 7, the protective cap 1000 may be detached from the case 100 by moving in one direction. At this time, the first sealing surface 1021 may move in one direction while compressing the first protrusion 1121, causing friction with the first protrusion 1121. Similarly, the second sealing surface 1022 may move in one direction while compressing the second protrusion 1122, causing friction with the second protrusion 1122. The protective cap 1000 may move sufficiently so that the first sealing surface 1021 and the second sealing surface 1022 may release contact with the first protrusion 1121 and the second protrusion 1122, respectively. If the protective cap 1000 moves further in one direction, the first sealing surface 1021 may face the first protrusion 1121. At this time, since the first sealing surface 1021 is farther from the surface 1130 of the sealing member 1100 than the second protrusion 1122, the second protrusion 1122 and the first sealing surface 1021 may not come into contact with each other. That is, during the process of detaching the protective cap 1000 from the case 100, the first sealing surface 1021 may not generate friction with the second protrusion 1122. Therefore, when a user detaches the protective cap 1000 from the case 100, the force burden that the user has to apply may be reduced.

The sealing member 1100 according to an example embodiment of the present disclosure may further include a band portion 1110. The band portion 1110 may have a band shape and be configured to cover around the outer circumference surface of the case 100. For example, the band portion 1110 may have a loop shape such as an elastic band. In this case, the case 100 may include a recess 210 for accommodating the band portion 1110. The recess 210 may have a shape corresponding to the outer peripheral surface of the band portion 1110 to engage with the band portion 1110. The band portion 1110 may be received in the recess 210 to be coupled to the case 100. The band portion 1110 may be formed from a different material than the case 100, allowing the band portion 1110 to be configured to be detachably coupled to the case 100. Alternatively, the band portion 1110 may be formed with the case 100 using a dual injection molding process. The first protrusion 1121 and the second protrusion 1122 may each be formed protruding outward from the band portion 1110.

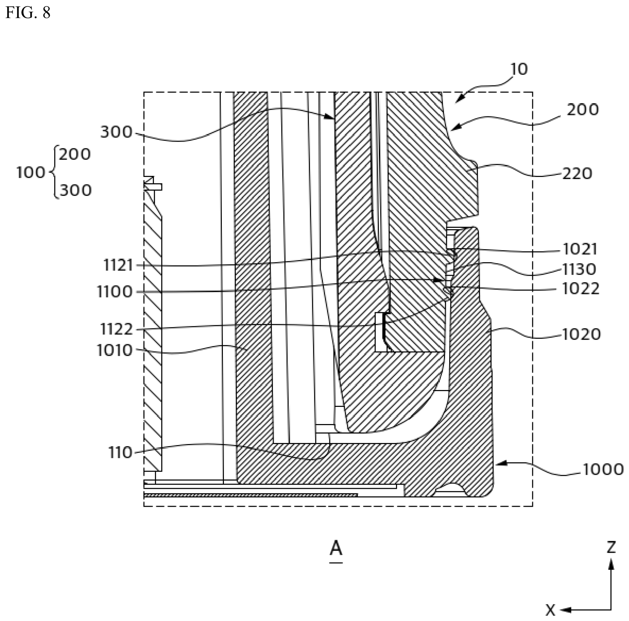

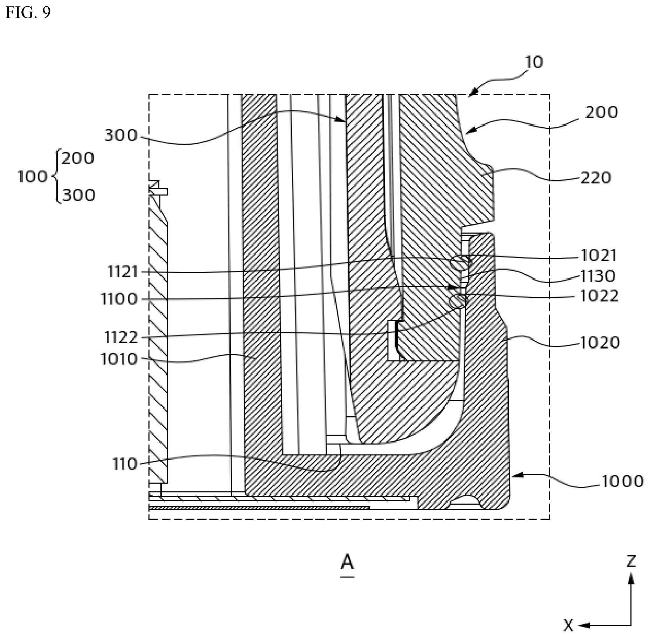

FIG. 8 is an exemplary diagram showing an applicator 10 according to another example embodiment of the present disclosure, enlarged from area A of FIG. 3, and FIG. 9 is an exemplary diagram showing an applicator 10 according to yet another example embodiment of the present disclosure, enlarging from area A of FIG. 3.

The sealing member 1100 may be formed in various shapes and materials, and the sealing member 1100 shown in FIGS. 4 to 7 may be only one of several examples. Hereinafter, with reference to FIGS. 8 to 9, another form of the sealing member 1100 is described.

Referring to FIG. 8, the sealing member 1100 may be formed from the same material as the case 100 and may be integrally formed with the case 100. For example, if the case 100 is manufactured from plastic, the sealing member 1100 may also be manufactured from plastic. However, even if the sealing member 1100 is formed integrally with the case 100 and made of the same plastic material, the plastic density of the sealing member 1100 and the case 100 may differ. The sealing member 1100 may be formed integrally with the case 100, with the first protrusion 1121 and the second protrusion 1122 formed to protrude from the outer surface of the case 100. The first protrusion 1121 and the second protrusion 1122 may also be formed of plastic and may be compressed to a predetermined degree by the pressing force of the first sealing surface 1021 and the second sealing surface 1022 to form a seal.

Referring to FIG. 9, the first protrusion 1121 and the second protrusion 1122 may be configured to be detachably coupled to the case 100. For example, the first protrusion 1121 and the second protrusion 1122 may have a ring-shaped configuration that covers around the outer peripheral surface of the case 100. The first protrusion 1121 and the second protrusion 1122 may be formed from a different material than the case 100. The first protrusion 1121 and the second protrusion 1122 may each be coupled to the case 100 in a separate form. In this case, a portion of the outer surface of the case 100 may be formed with a groove to accommodate at least a portion of the first protrusion 1121 and the second protrusion 1122. The first protrusion 1121 and the second protrusion 1122 have a circular cross-section, and the cross-sectional area of the first protrusion 1121 may be formed larger than the cross-sectional area of the second protrusion 1122. Therefore, the first protrusion 1121 and the second protrusion 1122 may form a step.

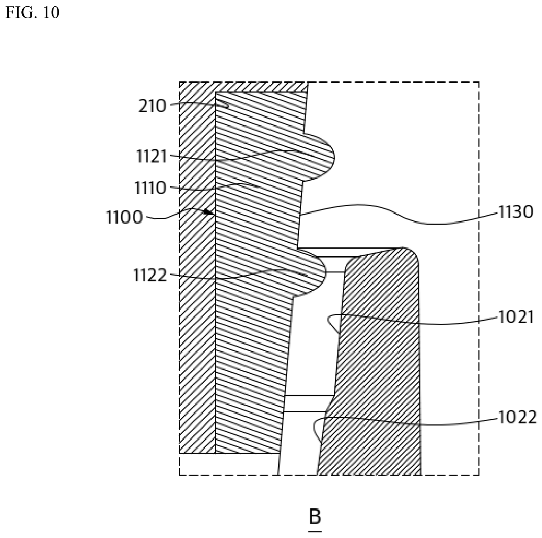

FIG. 10 is an illustrative diagram showing an applicator 10 according to yet another example embodiment of the present disclosure, enlarged from area B of FIG. 6.

Referring to FIG. 10 and FIG. 6 together, the surface 1130 of the sealing member 1100 according to yet another example embodiment of the present disclosure may be formed to be inclined. For example, the surface 1130 of the sealing member 1100 may be inclined toward the inside of the case 100 or the sealing member 1100 as it approaches the opening 110. In this case, it may be possible to form a step between the first protrusion 1121 and the second protrusion 1122 without differing the sizes of the first protrusion 1121 and the second protrusion 1122 themselves. The starting point of the second protrusion 1122 may be positioned relatively inward compared to the starting point of the first protrusion 1121.

The inner surface of the protective cap 1000 according to yet another example embodiment of the present disclosure may be formed to be inclined. For example, the inner surface of the protective cap 1000 facing the surface 1130 of the sealing member 1100 may be inclined together along the inclined surface 1130 of the sealing member 1100. The first sealing surface 1021 and the second sealing surface 1022 may be inclined toward the inside of the protective cap 1000.

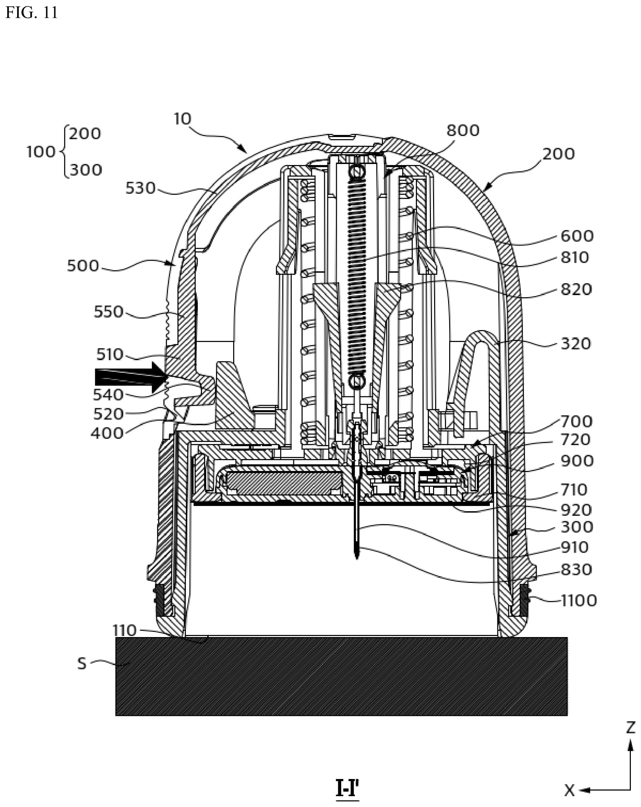

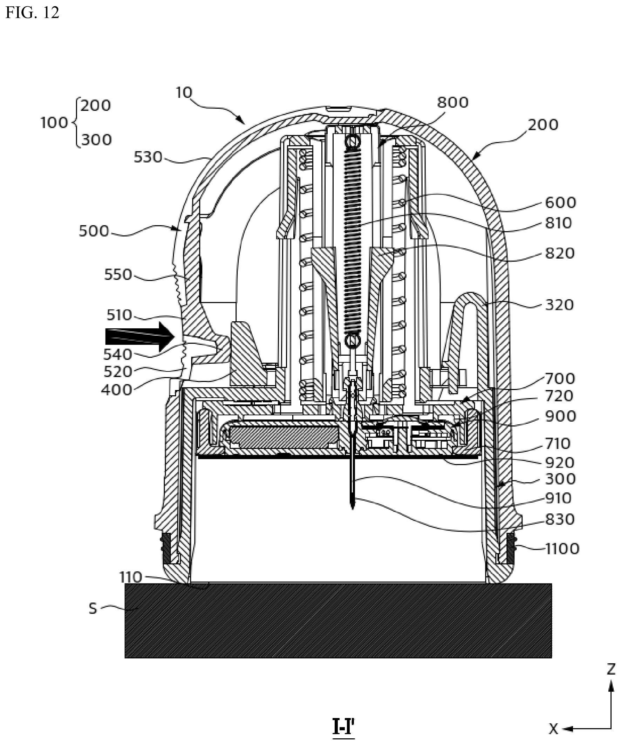

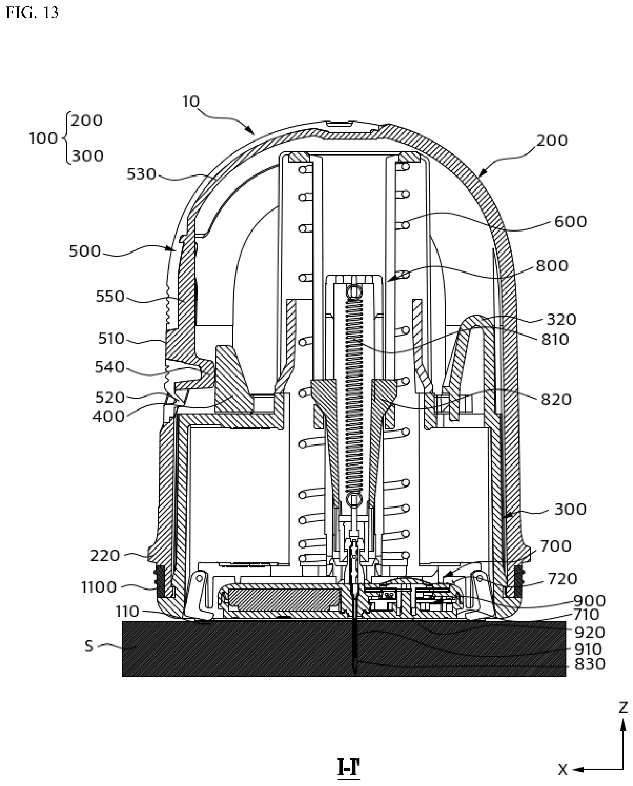

FIG. 11 is a cross-sectional view showing the state before triggering of an applicator 10 according to an example embodiment of the present disclosure, FIG. 12 is a cross-sectional view showing a button portion 510 of the applicator 10 in FIG. 10 pressed, and FIG. 13 is a cross-sectional view showing the state after triggering of the applicator 10 of FIG. 10.

Referring collectively to FIGS. 11 through 13 and FIG. 3, the case 100 according to an example embodiment of the present disclosure may include a main case 200 and an inner case 300. The main case 200 may be a component forming the outer shape, and the inner case 300 may be a component coupled to the inner side of the main case 200. The term "inner side" as used herein may refer to the internal space formed by the main case 200. The main case 200 may be a dome-shaped component having one side open, and the inner case 300 may be inserted and assembled through the open side. The inner case 300 may have an open surface on one side. For example, the inner case 300 may include the aforementioned opening 110. The sensor unit 900 is coupled to the case 100 in a separable manner and may be ejected through the opening 110 during use. The sensor unit 900 may be inserted and assembled through the opening 110 or ejected during the triggering stage.

The case 100 according to an example embodiment of the present disclosure may include an operating portion 500. The operating portion 500 may be a part that a user manipulates to operate the applicator 10. The operating portion 500 is joined to the case 100 and may seal an open side surface of the case 100. For example, the case 100 may include a button hole formed through at least a portion of the outer surface, allowing at least a portion of the operating portion 500 to move in and out when the user presses the button portion 510. The button hole may be formed by penetrating a portion of the outer surface of the main case 200. In this case, the operating portion 500 may seal the button hole.

The operating portion 500 according to an example embodiment of the present disclosure may include a pleated portion 520. The pleated portion 520 may be formed thinner than its surroundings. The pleated portion 520 may be formed thinner than other portions of the operating portion 500 connected to the pleated portion 520 to facilitate elastic deformation.

The operating portion 500 according to an example embodiment of the present disclosure may include a bonding cover 530. The bonding cover 530 may provide an area for bonding to the case 100.

The operating portion 500 according to an example embodiment of the present disclosure may include a push bar 540. The push bar 540 may protrude from the button portion 510 toward the interior space. The push bar 540 may protrude toward the interior space and contact a trigger 400. The trigger 400 is actuated by the push bar 540 and may release the stopped state of a plunger 700 so that the plunger 700 moves toward one side of the case 100 while being stopped adjacent to the center of the interior space.

When the button portion 510 according to an example embodiment of the present disclosure is pressed and moves toward the interior space side, the push bar 540 may also move toward the interior space side. The push bar 540 may push the trigger 400 toward the interior space side to release the stopped state of the plunger 700. That is, the push bar 540 may be arranged to press the trigger 400 toward the interior space side.

The push bar 540 according to an example embodiment of the present disclosure may be formed to reliably push the trigger 400. For example, the push bar 540 may be formed with a higher density than other parts of the operating portion 500. The operating portion 500 may be formed from an elastic material capable of elastic deformation and recovery as described above, and may be formed from a different material than the case 100. When the operating portion 500 is formed together with the case 100 using a dual injection molding process, sufficient time and pressure may be applied to the push bar 540 to form it with a high density. The push bar 540 is formed with high density and may possess sufficient rigidity to apply an external force to the trigger 400.

Additionally, the inner space side end of the push bar 540 according to an example embodiment of the present disclosure may be formed with a curved surface. Since the push bar 540 is formed from an elastic material, it may be capable of elastic deformation. The end of the push bar 540 formed as a curved surface may gradually increase the contact area with the trigger 400 during the process of applying an external force to the trigger 400.

The operating portion 500 according to an example embodiment of the present disclosure may include a support bar 550. The operating portion 500 is formed from an elastic material, and the button portion 510 may be formed thinly. Therefore, during the process of pressing the button, the button portion 510 may twist, causing the push bar 540 to deviate and fail to align with the trigger 400. The support bar 550 may be provided to stably hold the position of the push bar 540. The support bar 550 may extend upward from the push bar 540. The support bar 550 has a rod shape to sufficiently support the push bar 540 and may be connected to the inner side of the bonding cover 530 and the button portion 510.

The applicator 10 according to an example embodiment of the present disclosure may include the plunger 700. The plunger 700 may be disposed within the interior space of the case 100. Specifically, the plunger 700 may be disposed within the interior space of the inner case 300. At this time, a first elastic member 600 may be inserted between the plunger 700 and the inner case 300. The first elastic member 600 is inserted in a compressed state and may expand upon triggering to move the plunger 700.

The inner case 300 according to an example embodiment of the present disclosure may guide the movement of the plunger 700 toward the opening 110. For example, the inner case 300 may guide the movement of the plunger 700 in one direction. At least a portion of the inner shape of the inner case 300 may be formed to correspond with a portion of the outer shape of the plunger 700, and the inner case 300 may include a space allowing the plunger 700 to move toward the opening 110. The plunger 700 may be guided by at least a portion of the inner surface of the inner case 300 to move toward the opening 110.

The plunger 700 according to an example embodiment of the present disclosure may move the sensor unit 900 toward the opening 110. For example, the sensor unit 900 may move toward the opening 110. The sensor unit 900 may be configured to acquire the user's biometric information. The plunger 700 may be moved toward the opening 110 by the first elastic member 600, and in doing so, may move toward the opening 110 together with the sensor unit 900.

The applicator 10 according to an example embodiment of the present disclosure may further include the trigger 400. The trigger 400 may maintain the plunger 700 in a stationary state and release the plunger 700 from the stationary state during movement. The user may move the trigger 400 by pressing the button portion 510 of the aforementioned operating portion 500.

The applicator 10 according to an example embodiment of the present disclosure may include a needle assembly 800. The needle assembly 800 moves a needle 830 toward the opening 110 upon triggering, and the moved needle 830 may pierce a portion of the user's skin S. The needle assembly 800 may retract the needle 830 by moving it in the reverse direction of its initial movement after triggering, at a specific point. For example, the needle 830 may move toward the opening 110 to pierce a portion of the user's skin S, and then move in the reverse direction to be retracted.

In this specification, the plunger 700, sensor unit 900, needle assembly 800, and needle 830 are depicted as moving in an up-down direction in the drawings. However, this is merely an example and does not necessarily limit the direction of movement to the up-down direction. The direction may be appropriately modified as long as the needle 830 may pierce the user's skin S and the sensor unit 900 may be attached to the user's skin S. For example, although the opening 110 is open on one side of the case 100, the needle 830 may be triggered and moved in a direction intersecting the direction toward the opening 110 to pierce a portion of the user's skin S. Similarly, it is also possible for the direction in which the protective cap 1000 is attached to or detached from the case 100 and the direction in which the needle 830 moves to intersect each other.

The needle assembly 800 according to an example embodiment of the present disclosure may be inserted into the interior of the plunger 700. Specifically, the needle assembly 800 may be accommodated in a space provided by protruding from the upper portion of the plunger 700, and the sensor unit 900 may be positioned below the needle assembly 800.

FIG. 11 illustrates the applicator 10 in the state before triggering. Referring to FIG. 11, to use the applicator 10, the user may remove the protective cap 1000 and position the applicator 10 such that the opening 110 of the case 100 contacts the user's skin S.

The plunger 700 according to an example embodiment of the present disclosure may hold the sensor unit 900. A socket 720 is provided at the lower portion of the plunger 700 facing the opening 110, and the socket 720 forms a predetermined space to accommodate the sensor unit 900. At least one hook 710 may be formed around the socket 720. The hook 710 is bent at one end, and the bent portion of the hook 710 may support at least a portion of the sensor unit 900. The sensor unit 900 supported by the hook 710 may remain fixed within the socket 720.

The plunger 700 according to an example embodiment of the present disclosure may be in a stopped state near the central height of the case 100. At this time, the first elastic member 600 disposed between the plunger 700 and the inner case 300 may be in a compressed state. The trigger 400, positioned relatively to the left in the drawing prior to triggering, engages at least a portion of the plunger 700 and may secure the plunger 700 to prevent it from being moved by the first elastic member 600. That is, the trigger 400 may maintain the plunger 700 in a stationary state.

The sensor unit 900 according to an example embodiment of the present disclosure includes a probe 910 inserted into the body, in which the probe 910 may protrude toward one side. For example, the probe 910 may protrude toward the opening 110 in the state before triggering. An adhesive layer 920 may be formed on the lower portion of the body of the sensor unit 900. The adhesive layer 920 adheres to the human body, enabling the sensor unit 900 to be fixed to the skin S.

The needle assembly 800 according to an example embodiment of the present disclosure may be accommodated on the upper portion of the plunger 700. The needle 830 has a sharp tip capable of piercing the skin S and forms an internal cavity to accommodate the probe 910.

The user may position the applicator 10 at the application site and then press the button portion 510 of the operating portion 500 to trigger the applicator 10.

FIG. 12 shows the state with the button portion 510 pressed, and FIG. 13 shows the state after triggering. Referring to FIGS. 12 to 13, the plunger 700 moves toward the opening 110 to attach the sensor unit 900 to the skin S.

The user may press the button portion 510 toward the interior space side of the case 100. At this time, the push bar 540 located on the inner side of the button portion 510 may move toward the interior space side. One end of the push bar 540 presses the trigger 400, and the trigger 400 may move toward the interior space side. One surface of the trigger 400 may contact the push bar 540, and the opposite surface may contact the return member 320. The return member 320 is a component provided in the inner case 300 and may be elastically deformed to move the trigger 400 back to its original position. For example, when the user releases the button portion 510, the trigger 400 may be returned to its original position by the return member 320.

The trigger 400 moved toward the interior space may release the plunger 700 from its stopped state. The plunger 700 may move toward the opening 110 due to the expansion of the compressed first elastic member 600.

The sensor unit 900 and needle assembly 800 according to an example embodiment of the present disclosure may be moved toward the opening 110 together with the plunger 700.

The needle 830 of the needle assembly 800 according to an example embodiment of the present disclosure may move toward the opening 110 to pierce the skin S to a certain depth. The probe 910 located inside the needle 830 may be inserted into the hole formed in the skin S by the needle 830. The adhesive layer 920 of the sensor unit 900 attaches to the surface of the skin S, and the sensor unit 900 may be secured to the skin S with the probe 910 inserted into the skin S.

The inner case 300 according to an example embodiment of the present disclosure may have an inner wall that guides the movement of the hook 710 along the movement path of the hook 710. The inner wall of the inner case 300 may widen outward toward the opening 110. One surface of the hook 710 may move while in contact with the inner wall of the inner case 300. Upon reaching the end of the case 100, the hook 710 may rotate. Due to the rotation of the hook 710, the bent portion of the hook 710 that supported the lower part of the sensor unit 900 moves away from the sensor unit 900, and the hook 710 may release the fixation of the sensor unit 900.

The needle assembly 800 according to an example embodiment of the present disclosure may include a retrieval portion 820. One end of the retrieval portion 820 may secure the needle 830. The retrieval portion 820 may be secured by engaging a portion of the frame of the needle assembly 800. When the needle assembly 800 moves toward the opening 110 such that the needle 830 pierces the skin S to a certain depth, the retrieval portion 820 may be pressed inward by the inner case 300. At this time, the retrieval portion 820 may be released from engagement with the frame of the needle assembly 800.

The needle assembly 800 according to an example embodiment of the present disclosure may include a second elastic member 810. The second elastic member 810 may be coupled to the retrieval portion 820 in a tensioned state. When the retrieval portion 820 is released from engagement with the frame of the needle assembly 800, the second elastic member 810 pulls the retrieval portion 820 in the reverse direction of its movement.

The needle 830 may be retrieved by the retrieval portion 820 according to an example embodiment of the present disclosure in the reverse direction of its movement and accommodated within the needle assembly 800. The retrieved needle 830 is not exposed externally, thereby preventing injury to the user.

The mechanisms and configurations related to the triggering of the applicator 10 described above may be merely illustrative examples. Therefore, it is also possible to apply a different triggering mechanism to the applicator 10.

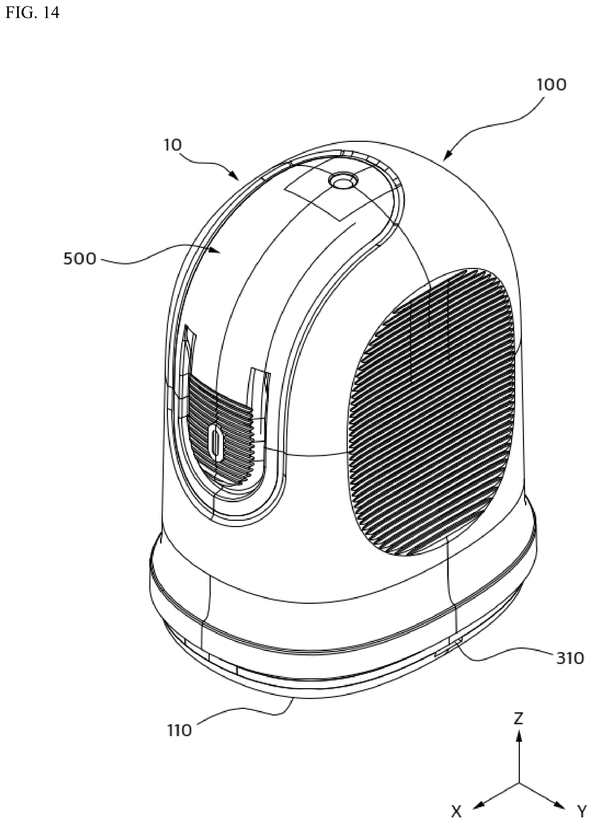

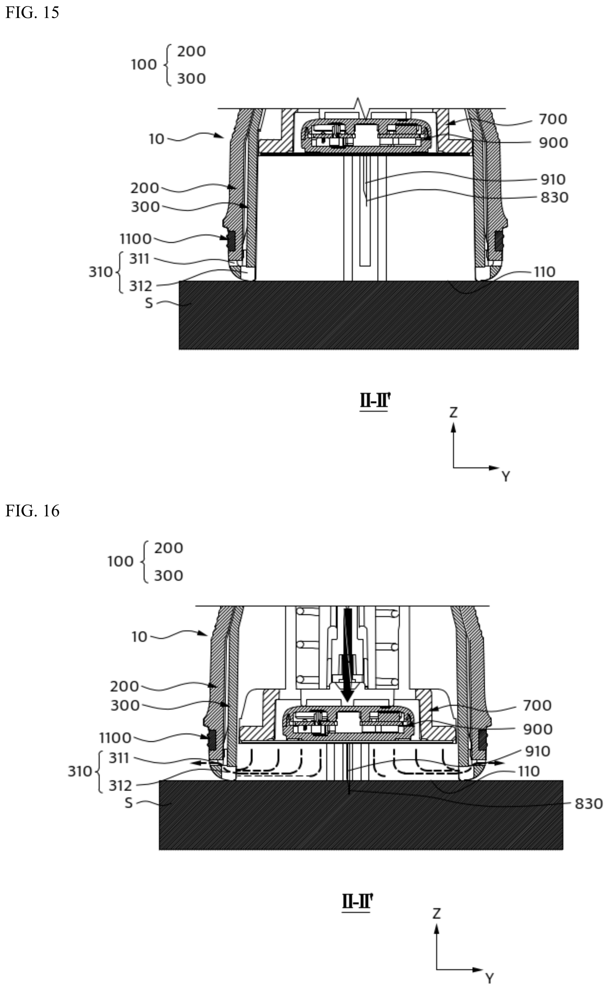

FIG. 14 is a perspective view showing the state of the applicator 10 according to an example embodiment of the present disclosure with the protective cap 1000 detached, FIG. 15 is a cross-sectional view of the applicator 10 according to an example embodiment of the present disclosure in the state before triggering, taken along section line II-II' of FIG. 1, and FIG. 16 is a cross-sectional view showing of the state after triggering of FIG. 14.

Referring to FIGS. 14 to 16, a vent 310 according to an example embodiment of the present disclosure may provide a path for air within the interior space of the case 100 to be discharged to the outside. Specifically, the vent 310 may provide a path for air pushed out by the plunger 700 moving toward the opening 110 during triggering to be discharged outside the case 100.

The vent 310 according to an example embodiment of the present disclosure may be spaced apart from the sealing member 1100 toward the opening 110. For example, the vent 310 may be more spaced apart from the sealing member 1100 in the direction toward the opening 110. The vent 310 is a hole provided to allow air from the interior space of the case 100 to be discharged to the outside and may communicate with the outside when the protective cap 1000 is detached. The interior space of the case 100 is required to be maintained in a sealed state until the applicator 10 is used, and the vent 310 may be sealed. Therefore, while the protective cap 1000 is attached to the case 100, the vent 310 may be positioned closer to the opening 110 than the sealing member 1100 to prevent communication with the outside.

In an example embodiment of the present disclosure, the vent 310 may include a first vent 311 and a second vent 312. The first vent 311 and the second vent 312 may form a stepped structure relative to each other. The first vent 311 and the second vent 312 may form a step difference. The first vent 311 and the second vent 312 may form a step difference toward the opening 110. The second vent 312 may be located closer to the opening 110 than the first vent 311 and may be positioned on the inner side. During use, at least one surface of the second vent 312 may contact the user's skin S and may be located at the end of the case 100. At least a portion of the second vent 312 may include a downwardly open configuration. Therefore, air expelled by the plunger 700 moving in one direction may be discharged to the outside through the second vent 312 until the end of the triggering state.

At least a portion of the vent 310 according to an example embodiment of the present disclosure may be formed between the opening 110 of the case 100 and the sealing member 1100. For example, the first vent 311 may be positioned between the end of the case 100 and the sealing member 1100. The first vent 311 is formed spaced apart from the end of the case 100, thereby preventing rigidity of one end of the case 100 from weakening.

The first vent 311 according to an example embodiment of the present disclosure may be a hole facing the outer side of the case 100. For example, the first vent 311 may have a form that opens radially based on the longitudinal direction of the case 100 when the main case 200 and the inner case 300 are combined.

The first vent 311 and the second vent 312 according to an example embodiment of the present disclosure may be connected to each other. For example, the first vent 311 and the second vent 312 may be connected to each other in a layered state. At least a portion of the first vent 311 may be located above at least a portion of the second vent 312 so that they communicate with each other.

According to example embodiments, it is possible to improve the sealing integrity of the applicator.

According to example embodiments, when a user detaches the protective cap of the applicator, the force burden required by the user may be reduced.

The above description has illustrated and explained the present disclosure in relation to a preferred embodiment for exemplifying the principles of the present disclosure. However, the present disclosure is not limited to the configuration and operation as illustrated and described herein. Rather, those skilled in the art will readily understand that numerous changes and modifications to the present disclosure are possible without departing from the spirit and scope of the appended claims.

## Claims

1. An applicator assembly (1) comprising:
a sensor unit (900) configured to be attached to a user's body to acquire the user's biometric information;
an applicator (10) comprising a case (100) forming an interior space with an opening (110) formed on one side, configured to attach the sensor unit (900) to the user's body; and
a protective cap (1000) configured to be coupled to the case (100) to shield the opening (110), wherein
the applicator (10) comprises a sealing member (1100) disposed adjacent to the opening (110) of the case (100) and configured to seal the interior space of the case (100) together with the protective cap (1000), and
the sealing member (1100) comprises a first protrusion (1121) and a second protrusion (1122) that protrude outward from a surface (1130) of the sealing member (1100) and form a step relative to each other.

2. The applicator assembly (1) of claim 1, wherein the protective cap (1000) covers at least a portion of an outer surface of the case (100) adjacent to the opening (110).

3. The applicator assembly (1) of claim 1 or 2, wherein the first protrusion (1121) is positioned farther from the opening (110) than the second protrusion (1122) and protrudes more than the second protrusion (1122).

4. The applicator assembly (1) of any one of claims 1 to 3, wherein the protective cap (1000) comprises a first sealing surface (1021) configured to compress the first protrusion (1121) and a second sealing surface (1022) configured to compress the second protrusion (1122) while being coupled to the case (100).

5. The applicator assembly (1) of claim 4, wherein the first sealing surface (1021) and the second sealing surface (1022) form a step relative to each other, and
a distance from the surface (1130) of the sealing member (1100) to the first sealing surface (1021) is greater than a distance from the surface (1130) of the sealing member (1100) to the second sealing surface (1022).

6. The applicator assembly (1) of claim 4 or 5, wherein the distance from the surface (1130) of the sealing member (1100) to the first sealing surface (1021) is greater than a height by which the second protrusion (1122) protrudes from the surface (1130) of the sealing member (1100).

7. The applicator assembly (1) of claim 6, wherein the first protrusion (1121) and the second protrusion (1122) comprise an elastic material capable of compressive deformation,
a height by which the first protrusion (1121) protrudes from the surface (1130) of the sealing member (1100) before compression is greater than the distance from the surface (1130) of the sealing member (1100) to the first sealing surface (1021), and
a height by which the second protrusion (1122) protrudes from the surface (1130) of the sealing member (1100) before compression is greater than the distance from the surface (1130) of the sealing member (1100) to the second sealing surface (1022).

8. The applicator assembly (1) of any one of the preceding claims, wherein the sealing member (1100) further comprises a band portion (1110) configured to cover around an outer surface of the case (100), and
the case (100) comprises a recess (210) configured to accommodate the band portion (1110).

9. The applicator assembly (1) of claim 8, wherein the band portion (1110) is configured to be detachably coupled to the case (100).

10. The applicator assembly (1) of any one of the preceding claims, wherein the sealing member (1100) is integrally formed with the case (100) from the same material as the case (100).

11. The applicator assembly (1) of any one of the preceding claims, wherein the first protrusion (1121) and the second protrusion (1122) are configured to be detachably coupled to the case (100) and have a ring shape that covers around an outer surface of the case (100).

12. The applicator assembly (1) of claim 11, wherein the first protrusion (1121) and the second protrusion (1122) have a circular cross-section, and a cross-sectional area of the first protrusion (1121) is formed to be larger than a cross-sectional area of the second protrusion (1122).

13. The applicator assembly (1) of any one of the preceding claims, wherein the surface (1130) of the sealing member (1100) is inclined toward an inside of the case (100) as approaching the opening (110).

14. The applicator assembly (1) of claim 13, wherein an inner surface of the protective cap (1000) facing the surface (1130) of the sealing member (1100) is inclined along the inclined surface (1130) of the sealing member (1100).

15. The applicator assembly (1) of any one of the preceding claims, wherein the sealing member (1100) is formed from a material capable of compressive deformation and comprises at least one of synthetic resin, rubber, or silicone.
